# EUROPÄISCHE PATENTSCHRIFT

(11) **EP 0 927 195 B1**
(45) Veröffentlichungstag und Bekanntmachung des Hinweises auf die Patenterteilung: **03.09.2003**
(21) Anmeldenummer: 97918834.9
(22) Anmeldetag: 10.09.1997
(51) Int. Cl.: C07K 14/76

(54) **VERFAHREN ZUR HERSTELLUNG EINES PLASMAPROTEIN-HÄLTIGEN ARZNEIMITTELS**
PROCESS FOR PRODUCING A PLASMA PROTEIN-CONTAINING MEDICAMENT
PROCEDE DE FABRICATION D'UN MEDICAMENT CONTENANT UNE PROTEINE PLASMATIQUE

(30) Priorität: 16.09.1996 AT 163396
(43) Veröffentlichungstag der Anmeldung: 07.07.1999
(73) Patentinhaber: Baxter Aktiengesellschaft, 1221 Wien (AT)
(72) Erfinder: TESCHNER, Wolfgang, A-1030 Wien (AT); LINNAU, Yendra, A-1224 Wien (AT); SVATOS, Sonja, A-2413 Berg (AT); IGEL, Herwig, A-1190 Wien (AT)
(74) Vertreter: Alge, Daniel, Mag. Dr. rer.nat.
(86) Internationale Anmeldenummer: AT9700197
(87) Internationale Veröffentlichungsnummer: WO98012225

(56) Entgegenhaltungen:
- EP-A- 0 484 464
- EP-A- 0 696 595

## Beschreibung

Die Erfindung betrifft ein Verfahren zur Herstellung eines Plasmaprotein-hältigen Arzneimittels, das im wesentlichen frei von unerwünschten Metallen ist, aus Zitratplasma oder einer zitrathältigen Plasmafraktion.

Humanes Albumin stellt mit einer Konzentration von 35-50 g/l die Hauptkomponente in Plasma dar. Seine therapeutische Verwendung ist seit langem bekannt, beispielsweise ist eine Verabreichung von Albumin bei akutem Blut- oder Plasmaverlust oder bei Versagen der vasomotorischen Regulation angezeigt. Da der osmotische Druck einer 20 %igen (25 %igen) Albuminlösung ungefähr das 4-fache (5-fache) des normalen menschlichen Serums beträgt, beruht die Wirkung von Albumin vorwiegend auf dessen Fähigkeit, den osmotischen Druck aufrechtzuerhalten.

Humanalbumin-Präparationen werden aus humanem Plasma durch mehrfache Fraktionierung, beispielsweise durch eine Fraktionierung nach Cohn, gewonnen oder mittels rekombinanter Verfahren hergestellt. Durch verschiedene Materialien während der Herstellung oder bei Lagerung der Albumin-Lösung in Glasbehältern gelangt Aluminium in die Albumin-Präparation, so daß der Endgehalt an Aluminium in den entsprechenden Präparationen beträchtlich sein kann.

Aluminium, welches eines der am meisten vorkommenden Elemente in der Natur darstellt, wurde in jüngster Zeit immer mehr in Zusammenhang mit unterschiedlichen Erkrankungen des menschlichen Körpers gebracht, vorwiegend mit Erkrankungen des Nerven- und Knochensystems. Während Lunge und Gastrointestinaltrakt eine effiziente Barriere für die Aufnahme von Aluminium bilden, ist diese Barriere bei Patienten, die intravenöse Präparate verabreicht bekommen, nicht mehr aufrecht, und das in den verabreichten Präparationen gegebenenfalls vorhandene Aluminium kann ungehindert aufgenommen werden. So berichteten beispielsweise D.S. Milliner et al. (N.Engl.J.Med. (1985), 312, S. 165-167), daß einige Albumin-Produkte mit großen Mengen an Aluminium zu Knochenerkrankungen oder Enzephalitis führten. Aluminium wird zunehmend auch in Zusammenhang mit der Alzheimer-Erkrankung gebracht.

Es wurden daher Anstrengungen unternommen, den Aluminium-Gehalt, beispielsweise von Albumin-Präparationen, niedrig zu halten. Aus der US-PS 5 372 997 ist beispielsweise ein Verfahren zur Reduktion des Aluminiumgehalts in Albumin-Präparationen durch Verwendung spezieller Glasbehälter einerseits und durch Behandlung mit einem Anionenaustauscher andererseits bekannt. Das Herauslösen des Aluminiums aus Glasbehältern wird durch die Verwendung eines speziellen, aluminiumarmen Glases und durch eine Dealkalisierung der inneren Oberfläche mit Ammoniumsulfatlösung oder mit schwefeliger Säure verhindert. Desweiteren wird eine Behandlung mit einem Kationenaustauscher durchgeführt. Zur Hitzebehandlung der Albuminlösung werden dann schließlich die hierfür gängigen Stabilisatoren, wie Natrium-N-Acetyltryptophan oder Natriumcaprylat zugesetzt.

Aus der EP-0 484 464-B1 ist ein Verfahren zum Reinigen eines Albumins von mehrwertigen Metallionen, die daran gebunden sind, bekannt, indem sie durch einwertige Metallionen, beispielsweise durch Ammonium- oder Alkalimetallionen ersetzt werden.

Auch aus der US-PS 5 250 663 ist ein Verfahren bekannt, nach dem ein im wesentlichen aluminiumfreies Albumin erhalten werden kann. Bei diesem Verfahren wird von einer Albumin-hältigen Fraktion, beispielsweise einer Cohn-Fraktion, ausgegangen, und zunächst werden verschiedene Fällungen durchgeführt. U.a. wird eine Hitze-Schockbehandlung in Gegenwart von Matriumcaprylat als Stabilisator und 10 bis 20 % Ethanol durchgeführt. Diese Lösung wird schließlich einer Ultrafiltration und einer Diafiltration unterworfen. Nach der Diafiltration sind Aluminium und andere Verunreinigungen entfernt, als Salze werden für die Diafiltration schwache Salzlösungen, wie 3 % NaCl, Natriumacetat oder in einigen Fällen Natriumcaprylat-Lösungen verwendet. Bei dieser Verwendung von Caprylat kommt es jedoch zu keinem Austausch von Salzen, da bereits vor der Diafiltration Caprylat als Stabilisator für die Hitzeschockbehandlung zugesetzt worden ist; es befinden sich also immer noch wesentliche Mengen an Zitrationen im Präparat.

Wie beispielsweise in J.C. May et al. (1992) (Vox Sang., 62, S. 65-69) beschrieben, spielt für die Aufnahme von Aluminium die Anwesenheit von Zitrationen, die eine hohe Affinität zu Aluminium aufweisen, eine wesentliche Rolle (siehe hierzu auch z.B. R.B. Martin (1986), J.Inorgan.Biochem., 28, S. 181-187), und dabei stellt die bloße Anwesenheit von Zitrationen bei sämtlichen Präparationen ein generelles Risiko für Metallionenkontaminationen dar.

In der EP-0 696 595-A1 werden bei einem Verfahren, bei welchem Caprylat zur Fraktionierung von Albumin verwendet wird, die Zitrationen im Zuge einer DEAE-Sephadex-Chromatographie entfernt. Hierbei kommt es jedoch zu keinem einfachen Austausch von Zitrationen gegen andere Ionen, sondern zu einer - aufgrund der hohen Kosten für die DEAE-Sephadex-Anionenaustauscher - aufwendigen Abtrennung von Zitrat.

Die vorliegende Erfindung stellt sich zur Aufgabe, ein neues und einfaches Verfahren zur Vermeidung bzw. Reduktion von unerwünschten Metallen in Plasmaprotein-hältigen Arzneimitteln zur Verfügung zu stellen, bei welchem sowohl die unerwünschten Metalle im Laufe des Herstellungsverfahrens entfernt werden, als auch eine Kontamination der fertiggestellten Präparate bei der Lagerung in metallhältigen Behältern verhindert bzw. reduziert wird.

Diese Aufgabe wird erfindungsgemäß durch ein Verfahren der eingangs erwähnten Art gelöst, welches die folgenden Schritte umfaßt:
- Austauschen des Zitrats und gegebenenfalls zitratgebundener Metalle in einer Plasmaprotein-hältigen Lösung gegen ein wasserlösliches Mono- oder Dicarboxylat bzw. eine organische Mono- oder Dicarbonsäure unter nicht-präzipitierenden Bedingungen,
- Gewinnen des Plasmaproteins oder der Plasmaproteine und
- Fertigstellen des Arzneimittels.

Überraschenderweise wurde gefunden, daß gerade die in der Plasmaprotein-hältigen Lösung enthaltenen Anionen entscheidend zur Entfernung der Metallkationen beitragen.

Im Gegensatz zu dem in der US-PS 5 250 663 beschriebenen Verfahren wird nicht nur Natriumcaprylat einer zitrathältigen Lösung bzw. einem zitrathältigen Niederschlag zugesetzt, sondern das Zitrat, welches einerseits gebundene Metallionen in komplexierter Form tragen kann als auch für die Herauslösung von unerwünschten Metallen im Rahmen des Herstellungsverfahrens bzw. bei der Lagerung des fertiggestellten Arzneimittels verantwortlich ist, wird durch ein wasserlösliches Mono- oder Dicarboxylat ersetzt.

Es hat sich nämlich gezeigt, daß, wenn Zitrationen-enthaltende Präparationen gefällt und danach in einem Zitrationen-freien Puffer aufgenommen werden, die neue Lösung immer noch erhebliche Anteile an Zitrationen enthalten kann. Da die meisten Fraktionierungsverfahren bei der Gewinnung von pharmazeutischen Präparationen aus Plasma - welchem fast ausnahmslos bei der Entnahme Zitrat zugegeben wird - ein oder mehrere Fällungsschritte aufweisen, stellt daher das erfindungsgemäße Austauschverfahren für Zitrationen eine interessante Möglichkeit für eine einfache, kostengünstige und effiziente Entfernung von Zitrationen dar, welche leicht in bereits etablierte Verfahrensabläufe einbezogen werden kann.

Als Plasmafraktion dient beispielsweise eine Fraktion, die nach der Cohn-Fraktionierung erhalten wird. Beispielsweise wird für die Herstellung von Albumin ein Albumin-hältiger Niederschlag der Cohn-Fraktionierung verwendet.

Um einen möglichst vollständigen Austausch des Zitrats herbeiführen zu können, muß daher der Austauschschritt unter nicht-präzipitierenden Bedingungen durchgeführt werden, da ansonsten - wie erwähnt - die Gefahr besteht, daß das Zitrat durch seine hohe Affinität zum gefällten Protein nur unzureichend entfernt werden kann. Bevorzugterweise erfolgt der Austausch des Zitrats zu einem frühen Zeitpunkt im Herstellungsverfahren.

Gemäß einer bevorzugten Ausführungsform des erfindungsgemäßen Verfahrens wird als Plasmaprotein-hältiges Arzneimittel ein Arzneimittel, enthaltend ein oder mehrere Faktoren der Gerinnung und Fibrinolyse, Immunglobuline, Glykoproteine und/oder Albumin, hergestellt. Als Gerinnungs- und Fibrinolysefaktoren kommen dabei insbesondere Fibrinogen, Prothrombin, die Faktoren V, VII, VIII, IX, X, XI, XII und XIII, gegebenenfalls in deren aktivierter Form, von Willebrand-Faktor, aber auch Antikoagulantien, wie Heparin, Heparinoide oder Cumarinderivate, oder Fibrinolytika, wie Streptokinase, Urokinase, Pro-Urokinase, t-PA oder Plasmin in Betracht. Als Immunglobuline können verschiedene Präparate, enthaltend Immunglobuline der Klassen IgG, IgA, IgM und Gemische derselben, gegebenenfalls in hohen Titern, hergestellt werden.

Als Glykoprotein kommt beispielsweise Orosomucoid in Frage.

Bevorzugterweise wird zum Austauschen des Zitrats ein Salz einer organischen Carbonsäure mit 2 bis 20 Kohlenstoffatomen verwendet, wobei ein Caprylat oder ein Tartrat oder Gemische davon besonders bevorzugt werden.

Für die Zwecke der vorliegenden Erfindung ist auch eine organische Mono- oder Dicarbonsäure als Mono- oder Dicarboxylat zu verstehen, da der Austausch des Zitrats in Lösung ohnehin immer gegen das Säureanion erfolgt. Bevorzugterweise wird zum Austauschen des Zitrats eine organische Mono- oder Dicarbonsäure mit 2 bis 4 Kohlenstoffatomen verwendet.

Das erfindungsgemäße Verfahren hat sich als ganz besonders geeignet zur Herstellung von Plasmaprotein-hältigen Arzneimitteln erwiesen, die speziell im Hinblick auf ihre Aluminium-Kontamination hervorragende Eigenschaften aufweisen, indem sie im wesentlichen frei von jeglichem detektierbaren Aluminium sind.

Wie erwähnt, muß das Austauschen des Zitrats unter nicht-präzipitierenden Bedingungen erfolgen. Vorzugsweise wird der Austauschschritt während einer Diafiltration, Ultrafiltration bzw. während eines chromatographischen Verfahrens vorgenommen, da sich diese Schritte als besonders geeignet für einen einfachen, kostengünstigen und effizienten Austausch herausgestellt haben.

Die Bedingungen während des Austauschschrittes hängen von dem verwendeten Verfahren ab und werden insbesondere so gewählt, daß der Austausch des Zitrats und gegebenenfalls auch zitratgebundener Metalle möglichst vollständig erfolgt. Deshalb sind entsprechende verfahrensbestimmende Parameter, insbesondere Temperatur, Dauer des Austauschschrittes und Konzentration an jeweiligem Mono- oder Dicarboxylat bzw. an Mono- oder Dicarbonsäure, zu optimieren.

Vorzugsweise liegt die Temperatur während des Austausches in einem Bereich zwischen 0 und 50°C, weiters bevorzugt in einem Bereich zwischen 10 und 30°C, am meisten bevorzugt bei etwa Raumtemperatur. Die jeweilige Zeitspanne für den Austausch ist insbesondere von dem Verhältnis von auszutauschendem Volumen zu Membranoberfläche und von der Temperatur abhängig und beträgt vorzugsweise mindestens 30 Minuten, insbesondere liegt die Zeitspanne in einem Bereich zwischen 30 Minuten und mehreren Stunden.

Im allgemeinen ist ein Parameter wie die Zeitspanne gerade auch von dem jeweiligen Austauschvolumen des entsprechenden Materials abhängig. Vorzugsweise beträgt das Austauschvolumen mindestens das 5-fache, am meisten bevorzugt mindestens das 30-fache der Ausgangslösung, und die Zeitspanne für den Austausch wird dementsprechend bemessen.

Die Konzentration an Mono- oder Dicarboxylat bzw. Mono- oder Dicarbonsäure liegt vorzugsweise in einem Bereich zwischen 0,001 und 10 mol/l, am meisten bevorzugt in einem Bereich zwischen 0,001 und 1 mol/l.

Beispielsweise wird Natriumcaprylat in einer Konzentration zwischen 1,0 mmol/l und 1,5 mol/l zugegeben, vorzugsweise in einem Bereich zwischen 1,0 mmol/l und 25 mmol/l.

Natriumacetat wird beispielsweise in einer Konzentration zwischen 1 mmol/l und 5,5 mol/l, vorzugsweise zwischen 50 mmol/l und 1,0 mol/l, zugegeben.

Das Natriumsalz der Hexansäure wird beispielsweise in einer Konzentration zwischen 1,0 mmol/l und 1,0 mol/l, vorzugsweise in einem Bereich zwischen 5,0 mmol/l und 0,1 mol/l, zugesetzt.

Natriumtartrat kann in einer Konzentration zwischen 1,0 mmol/l und 1,2 mol/l, vorzugsweise zwischen 10,0 mmol/l und 0,2 mol/l, zugesetzt werden.

Für Salze höherer Säuren sind also die effizienten Mengen bereits im Bereich von 0,001 bis 0,1 mol/l zu finden, während Salze niedrigerer Säuren vorzugsweise in etwas höherer Konzentration zugesetzt werden.

Ein weiterer verfahrensbestimmender Parameter ist der pH-Wert der Lösung. Dieser liegt vorzugsweise bei pH 6 bis 8, am meisten bevorzugt bei pH 6,5 bis 7,5.

In der Lösung können neben Carboxylat bzw. Carbonsäure auch anorganische Salze, wie z.B. Natrium- oder Kalium-Salze, zur Erhöhung der Ionenstärke enthalten sein. Beispielsweise ist eine mindestens 4 %ige Natriumchloridlösung enthalten. Desweiteren können verschiedene Puffersalze enthalten sein.

Als Materialien für das jeweilige Austauschverfahren kommen insbesondere kommerziell erhältliche Materialien, wie z.B. Diafiltrationsmembranen, Ultrafiltrationseinheiten, diverse chromatographische Gele, Molekularsiebe und weitere, in Frage. Alle diese Materialien können auf organischer oder anorganischer Basis sein; sie können synthetischen oder biologischen Ursprungs sein.

Es zeigte sich, daß das erfindungsgemäße Verfahren dann besonders vorteilhaft ist, wenn die Plasmaprotein-hältige Lösung vor dem Austauschen gereinigt und/oder ankonzentriert wird.

Wie bei allen Arzneimitteln, welche auf Plasma als Rohstoff basieren, sollten auch im Rahmen des erfindungsgemäßen Herstellungsverfahrens ein oder mehrere Schritte zur Inaktivierung gegebenenfalls vorhandener Viren vorgesehen werden.

Eine bevorzugte Ausführungsform des erfindungsgemäßen Verfahrens betrifft daher ein Verfahren, bei welchem die Plasmaprotein-hältige Lösung vor und/oder nach dem Austauschen zur Inaktivierung gegebenenfalls vorhandener Viren behandelt, vorzugsweise hitzebehandelt, wird. Gängige Virus-Inaktivierungshandlungen, welche im Rahmen des vorliegenden Verfahrens eingesetzt werden können, sind in der EP-0 159 311, der EP-0 519 901 oder der EP-0 674 531 beschrieben.

Besonders günstig ist, wenn vor einer Virus-Inaktivierungsbehandlung das gewonnene Plasmaprotein noch einer Dialyse gegen ein salzarmes Mittel, z.B. Wasser, unterworfen wird. Hierbei kann immer noch ein zusätzlicher stabilisierender Effekt für das Plasmaprotein auf Grund der Gegenwart des Mono- oder Dicarboxylats gegeben sein. Das Gewinnen des Plasmaproteins oder der Plasmaproteine und das Fertigstellen des Arzneimittels sollten vorzugsweise ausschließlich mit zitratfreien Komponenten vorgenommen werden, um die erneute Kontamination des Präparats mit Zitrationen, welche bei längerer Lagerung für eine erneute Kontamination mit Metallionen verantwortlich sind, zu vermeiden.

Das erfindungsgemäße Verfahren hat sich zwar als ganz besonders geeignet zur Entfernung von Aluminiumionen bzw. zur Verhinderung einer erneuten Kontamination mit Aluminiumionen während der Lagerung der Arzneimittel herausgestellt. Gleichwohl können aber auch andere Metallionen, von welchen bekannt ist, daß sie Plasmaprotein-hältige Arzneimittel kontaminieren können, wie z.B.

Aluminium-ähnliche Metalle, Cadmium, Zink, Blei, Eisen, und weitere, effizient reduziert werden.

Ein Plasmaprotein-hältiges Arzneimittel, dass nach dem erfindungsgemäßen Verfahren erhältlich ist, kann einen Gehalt an Aluminium von weniger als 10 µg/l, insbesondere von weniger als 200 ng/l, ermittelt beispielsweise durch Atomabsorptions-Spektroskopie, aufweisen, wobei dieser Höchstgehalt auch nach längerer Lagerung, sogar bei Lagerung von über 5 Jahren, nicht überschritten wird.

Das erfindungsgemäße Plasmaprotein-hältige Arzneimittel kann in den verschiedensten aus dem Stand der Technik bekannten Behältern (Containern) gelagert werden. Derartige Behälter können aus Glas, Kunststoff, Metallen oder Kombinationen davon bestehen. Die Behälter können auch speziell vorbehandelt sein, so kann beispielsweise die Oberfläche silikonisiert sein. Als Gläser können sowohl Hartgläser als auch Weichgläser (siehe beispielsweise Gläser, Klassifikation USP 23, S. 1781) verwendet werden.

Insbesondere weist das erfindungsgemäße Plasmaprotein-hältige Arzneimittel auch bei Lagerung in Hartgläsern einen Gehalt an Aluminium von weniger als 10 µg/l, insbesondere weniger als 200 ng/l, auf.

Die vorliegende Erfindung wird anhand der folgenden Beispiele, auf die sie jedoch nicht eingeschränkt sein soll, näher erläutert.

### Beispiel 1:

Ein Niederschlag der Cohn-Fraktionierung, der Albumin in einer Reinheit von >95 % enthält, wird 1+2 (G/V; 1 kg in 2 l) in 50 g/l NaCl-Lösung bei neutralem pH-Wert gelöst. Die Lösung wird bei +4°C kontinuierlich gegen Wasser mit einer regenerierten Zellulosemembran diafiltriert. Dabei wird 0,1 mmol Caprylat pro g Protein zugesetzt.

In der nachstehenden Tabelle 1 a) sind die Aluminiumabreicherung und die Zitratabreicherung nach der Diafiltration ohne Zusatz eines Carboxylats, wie beispielsweise Caprylat, Tartrat, Salz der Hexansäure oder eines Acetats dargestellt.

### a) ohne Zugabe eines Carboxylats

**TABELLE 1:**

| | Aluminiumgehalt | | Zitratgehalt | |
|---|---|---|---|---|
| Probe | µg/g Protein | in % | µmol/g Protein | in % |
| vor Diafiltration | 2,802 | 100,0 | 166 | 100,0 |
| Diakonzentrat | 0,704 | 25,1 | 8,6 | 5,2 |

Die folgende Tabelle 1 b) zeigt die Aluminiumabreicherung und die Zitratabreicherung nach der Diafiltration mit Zusatz von 0,1 mmol Caprylat.

### 1 b) mit Zugabe von Caprylat

| | Aluminiumgehalt | | Zitratgehalt | |
|---|---|---|---|---|
| Probe | µg/g Protein | in % | µmol/g Protein | in % |
| vor Diafiltration | 2,999 | 100,0 | 139,5 | 100,0 |
| Diakonzentrat | 0,096 | 3,2 | 1,1 | 0,8 |

Ein Vergleich dieser Tabelle mit der Tabelle 1 a), also den entsprechenden Abreicherungen ohne Zusatz eines Caprylats, zeigt deutlich, daß mit einer 0,1 mmol Caprylatzugabe pro g Protein eine deutlich höhere Abreicherung an Zitrat und Aluminium zu erreichen ist, als dies ohne Zugabe eines Carboxylats, wie beispielsweise von Caprylat, der Fall ist.

### Beispiel 2:

Ein Albumin-hältiger Niederschlag wird wie in Beispiel 1 beschrieben gelöst und anschließend diafiltriert. Dabei werden 0,5 mmol eines Natriumsalzes der Hexansäure pro g Protein zugesetzt. Nach Abschluß der Diafiltration ergeben sich folgende Aluminiumund Zitratabreicherungen (Tabelle 2).

**TABELLE 2:**

| | Aluminiumgehalt | | Zitratgehalt | |
|---|---|---|---|---|
| Probe | µg/g Protein | in % | µmol/g Protein | in % |
| vor Diafiltration | 3,368 | 100,0 | 135 | 100,0 |
| Diakonzentrat | 0,127 | 3,8 | 1,8 | 1,3 |

Wie aus dieser Tabelle im Vergleich zu Tabelle 1 a) deutlich hervorgeht, ist mit einer Zugabe von 0,5 mmol eines Natriumsalzes der Hexansäure pro g Protein eine deutlich höhere Abreicherung an Zitrat und Aluminium zu erreichen, als dies ohne Zugabe des Hexansäuresalzes der Fall ist.

### Beispiel 3:

Ein Niederschlag der Cohn-Fraktionierung, der Albumin in einer Reinheit von >95 % enthält, wird 1+2 (G/V; 1 kg in 2 l) in 50 g/l NaCl-Lösung bei neutralem pH-Wert gelöst. Die Lösung wird bei +4°C kontinuierlich gegen Wasser mit einer regenerierten Zellulosemembran diafiltriert. Dabei werden 5 mmol Acetat pro g Protein zugesetzt.

In der nachstehenden Tabelle 3 sind die Aluminiumabreicherung und die Zitratabreicherung nach der Diafiltration mit Zusatz von 5 mmol Acetat pro g Protein dargestellt.

**TABELLE 3:**

| | Aluminiumgehalt | | Zitratgehalt | |
|---|---|---|---|---|
| Probe | µg/g Protein | in % | µmol/g Protein | in % |
| vor Diafiltration | 3,95 | 100,0 | 155 | 100,0 |
| Diakonzentrat | 0,3 | 7,6 | 2,6 | 1,7 |

Wie aus dieser Tabelle im Vergleich zu Tabelle 1 a) deutlich hervorgeht, ist mit einer Zugabe von 5 mmol Acetat pro g Protein eine deutlich höhere Abreicherung an Zitrat und Aluminium zu erreichen, als dies ohne Zugabe des Acetats der Fall ist.

### Beispiel 4:

Ein Albumin-hältiger Niederschlag wird wie in Beispiel 1 beschrieben gelöst und anschließend diafiltriert. Dabei wird 1,0 mmol Tartrat pro g Protein zugesetzt. Nach Abschluß der Diafiltration ergeben sich folgende Aluminium- und Zitratabreicherungen (Tabelle 4).

**TABELLE 4:**

| | Aluminiumgehalt | | Zitratgehalt | |
|---|---|---|---|---|
| Probe | µg/g Protein | in % | µmol/g Protein | in % |
| vor Diafiltration | 6,29 | 100,0 | 129 | 100,0 |
| Diakonzentrat | 0,62 | 9,9 | 1,0 | 0,8 |

Wie aus dieser Tabelle im Vergleich zu Tabelle 1 a) deutlich hervorgeht, ist mit einer Zugabe von 1,0 mmol Tartrat pro g Protein eine deutlich höhere Abreicherung an Zitrat und Aluminium zu erreichen, als dies ohne Zugabe von Tartrat der Fall ist.

## Patentansprüche

1. Verfahren zur Herstellung eines Plasmaprotein-hältigen Arzneimittels, das im wesentlichen frei von unerwünschten Metallen ist und auch bei Lagerung in metallhältigen Behältern keine Metalle aufnimmt, aus Zitratplasma oder einer zitrathältigen Plasmafraktion, welches Verfahren die folgenden Schritte umfaßt:
- Austauschen des Zitrats und gegebenenfalls zitratgebundener Metalle in einer Plasmaprotein-hältigen Lösung gegen ein wasserlösliches Mono- oder Dicarboxylat bzw. eine organische Mono- oder Dicarbonsäure unter nicht-präzipitierenden Bedingungen,
- Gewinnen des Plasmaproteins oder der Plasmaproteine und
- Fertigstellen des Arzneimittels.

2. Verfahren nach Anspruch 1, **dadurch gekennzeichnet, daß** als Plasmaprotein-hältiges Arzneimittel ein Arzneimittel, enthaltend ein oder mehrere Plasmaproteine, ausgewählt aus der Gruppe bestehend aus den Faktoren der Gerinnung und Fibrinolyse, Immunglobulinen, Glykoproteinen und Albumin, hergestellt wird.

3. Verfahren nach Anspruch 1 oder 2, **dadurch gekennzeichnet, daß** zum Austauschen des Zitrats ein Salz einer organischen Carbonsäure mit 2 bis 20 Kohlenstoffatomen verwendet wird.

4. Verfahren nach einem der Ansprüche 1 bis 3, **dadurch gekennzeichnet, daß** zum Austauschen des Zitrats ein Caprylat und/oder ein Tartrat verwendet wird.

5. Verfahren nach einem der Ansprüche 1 bis 3, **dadurch gekennzeichnet, daß** zum Austauschen des Zitrats eine organische Monooder Dicarbonsäure mit 2 bis 4 Kohlenstoffatomen verwendet wird.

6. Verfahren nach einem der Ansprüche 1 bis 5, **dadurch gekennzeichnet, daß** ein Plasmaprotein-hältiges Arzneimittel, das im wesentlichen frei von Aluminium ist, hergestellt wird.

7. Verfahren nach einem der Ansprüche 1 bis 6, **dadurch gekennzeichnet, dass** das Austauschen des Zitrats während einer Diafiltration, Ultrafiltration, einer Gelpermeationschromatographie bzw. eines chromatographischen Trennverfahrens vorgenommen wird, welche die Abtrennung des Proteins von Salzen erlauben.

8. Verfahren nach einem der Ansprüche 1 bis 7, **dadurch gekennzeichnet, dass** die Plasmaprotein-hältige Lösung vor dem Austauschen gereinigt und/oder ankonzentriert wird.

9. Verfahren nach einem der Ansprüche 1 bis 8, **dadurch gekennzeichnet, dass** die Plasmaprotein-hältige Lösung vor und/oder nach dem Austauschen zur Inaktivierung gegebenenfalls vorhandener Viren behandelt, vorzugsweise hitzebehandelt, wird.

10. Verfahren nach einem der Ansprüche 1 bis 9, **dadurch gekennzeichnet, dass** die Virusinaktivierungsbehandlung unmittelbar nach dem Gewinnen des Plasmaproteins in Gegenwart des Mono- oder Dicarboxylats durchgeführt wird.

11. Verfahren nach einem der Ansprüche 1 bis 10, **dadurch gekennzeichnet, dass** das Fertigstellen des Arzneimittels ausschließlich mit Zitrat-freien Komponenten vorgenommen wird.

12. Verfahren nach einem der Ansprüche 1 bis 11, **dadurch gekennzeichnet, dass** das Austauschen des Zitrats in Gegenwart von Natriumchlorid, vorzugsweise mit einer mindestens 4 Gew.%-igen Natriumchloridlösung, vorgenommen wird.

## Claims

1. A method of preparing a plasma-protein-containing medicament from citrated plasma or from a citrate-containing plasma fraction, which medicament is substantially free from undesired metals and also does not take up any metals when stored in metal-containing containers, which method comprises the following steps:
- exchanging the citrate and optionally citrate-bound metals in a plasma-protein-containing solution for a water-soluble mono- or dicarboxylate or for an organic mono- or dicarboxylic acid under non-precipitating conditions,
- recovering the plasma protein or the plasma proteins, and
- finishing the medicament.

2. A method according to claim 1, **characterized in that** as the plasma-protein-containing medicament, a medicament comprising one or several plasma proteins selected from the group consisting of the factors of coagulation and fibrinolysis, immunoglobulins, glycoproteins and albumin is prepared.

3. A method according to claim 1 or 2, **characterized in that** a salt of an organic carboxylic acid having 2 to 20 carbon atoms is used for exchanging the citrate.

4. A method according to any one of claims 1 to 3, **characterized in that** a caprylate and/or a tartrate is used for exchanging the citrate.

5. A method according to any one of claims 1 to 3, **characterized in that** an organic mono- or dicarboxylic acid having 2 to 4 carbon atoms is used for exchanging the citrate.

6. A method according to any one of claims 1 to 5, **characterized in that** a plasma-protein-containing medicament which is substantially free from aluminum is prepared.

7. A method according to any one of claims 1 to 6, **characterized in that** exchanging of the citrate is effected during a diafiltration, ultrafiltration, a gel permeation chromatography or a chromatographic separation method, respectively, which enable the separation of the protein from salts.

8. A method according to any one of claims 1 to 7, **characterized in that** before the exchange, the plasma-protein-containing solution is purified and/or concentrated.

9. A method according to any one of claims 1 to 8, **characterized in that** the plasma-protein-containing solution is treated, preferably heat-treated, before and/or after the exchange, so as to inactivate possibly present viruses.

10. A method according to any one of claims 1 to 9, **characterized in that** the virus inactivation treatment is effected immediately after the recovery of the plasma protein in the presence of the mono- or dicarboxylate.

11. A method according to any one of claims 1 to 10, **characterized in that** finishing of the medicament is effected exclusively with citrate-free components.

12. A method according to any one of claims 1 to 11, **characterized in that** the exchange of the citrate is effected in the presence of sodium chloride, preferably with an at least 4 % by weight sodium chloride solution.

## Revendications

1. Procédé de fabrication d'un médicament contenant une protéine plasmatique, globalement exempt de métaux indésirables et n'absorbant pas les métaux en cas de stockage dans des emballages contenant des métaux, à partir de plasma recueilli sur citrate ou d'une fraction plasmatique contenant du citrate, ledit procédé comprenant les étapes suivantes :
- échange du citrate et le cas échéant de métaux liés au citrate dans une solution contenant une protéine plasmatique contre un monocarboxylate ou un dicarboxylate hydrosolubles - ou un acide monocarboxylique ou un acide oxalique organiques dans des conditions n'entraînant pas de précipitation,
- récupération de la protéine plasmatique ou des protéines plasmatique et
- achèvement du médicament.

2. Procédé selon la revendication 1 **caractérisé en ce que** comme médicament contenant une protéine plasmatique est fabriqué un médicament contenant une ou plusieurs protéines plasmatiques choisies dans le groupe comprenant les facteurs de coagulation et de fibrinolyse, les immunoglobulines, des glycoprotéines et l'albumine.

3. Procédé selon la revendication 1 ou 2 **caractérisé en ce que**, pour l'échange du citrate, on utilise un sel ou un acide carboxylique organique avec 2 à 20 atomes de carbone.

4. Procédé selon l'une des revendications 1 à 3 **caractérisé en ce que**, pour l'échange du citrate, on utilise un caprylate et/ou un tartrate.

5. Procédé selon l'une des revendications 1 à 3 **caractérisé en ce que**, pour l'échange du citrate, on utilise un acide monocarboxylique ou un acide oxalique organiques avec 2 à 4 atomes de carbone.

6. Procédé selon l'une des revendications 1 à 5 **caractérisé en ce qu'**un médicament contenant une protéine plasmatique globalement exempt d'aluminium est fabriqué.

7. Procédé selon l'une des revendications 1 à 6 **caractérisé en ce que** l'échange du citrate a lieu au cours d'une diafiltration, d'une ultrafiltration, d'une chromatographie par perméation de gel ou d'un procédé de séparation par chromatographie permettant de séparer la protéine des sels.

8. Procédé selon l'une des revendications 1 à 7 **caractérisé en ce que** la solution contenant la protéine plasmatique est purifiée et/ou concentrée avant l'échange.

9. Procédé selon l'une des revendications 1 à 8 **caractérisé en ce que** la solution contenant la protéine plasmatique est traitée, de préférence à la chaleur, avant et/ou après l'échange pour inactiver des virus éventuellement présents.

10. Procédé selon l'une des revendications 1 à 9 **caractérisé en ce que** le traitement de désactivation des virus est réalisé immédiatement après récupération de la protéine plasmatique en présence du monocarboxylate ou du dicarboxylate.

11. Procédé selon l'une des revendications 1 à 10 **caractérisé en ce que** l'achèvement du médicament est finalement réalisé avec des composants sans citrate.

12. Procédé selon l'une des revendications 1 à 11 **caractérisé en ce que** l'échange du citrate est réalisé en présence de chlorure de sodium, de préférence avec une solution de chlorure de sodium à au moins 4 %.
